(19) Europäisches Patentamt
European Patent Office
Office européen des brevets

(11) Veröffentlichungsnummer: **0 290 741 B1**

# (12) EUROPÄISCHE PATENTSCHRIFT

(45) Veröffentlichungstag der Patentschrift: **09.10.91**

(51) Int. Cl.[5]: **A61K 7/48**, C08B 37/08

(21) Anmeldenummer: **88103769.1**

(22) Anmeldetag: **10.03.88**

## (54) Neue makromolekulare, oberflächenaktive, quaternäre, N-substituierte Chitosanderivate sowie kosmetisches Mittel auf der Basis dieser neuen Chitosanderivate.

(30) Priorität: **09.05.87 DE 3715576**

(43) Veröffentlichungstag der Anmeldung:
**17.11.88 Patentblatt 88/46**

(45) Bekanntmachung des Hinweises auf die Patenterteilung:
**09.10.91 Patentblatt 91/41**

(84) Benannte Vertragsstaaten:
**AT BE CH DE ES FR GB GR IT LI NL SE**

(56) Entgegenhaltungen:
**EP-A- 0 115 574**
**EP-A- 0 192 925**

**PATENT ABSTRACTS OF JAPAN, Band 10, Nr. 226 (C-364)[2282], 7. August 1986; & JP-A-61 60 701 (LION CORP.) 28-03-1986**

(73) Patentinhaber: **Wella Aktiengesellschaft**
**Berliner Allee 65**
**W-6100 Darmstadt(DE)**

(72) Erfinder: **Lang, Günther, Dr.**
**Auf der Roten Erde 10B**
**W-6107 Reinheim 5(DE)**
Erfinder: **Wendel, Harald**
**Grabengasse 3**
**W-6105 Ober-Ramstadt(DE)**

**EP 0 290 741 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein kosmetisches Mittel zur Behandlung von Haaren und der Haut mit einem Gehalt an neuen makromolekularen, oberflächenaktiven, quaternären, vom Chitosan abgeleiteten, N-substituierten Verbindungen, welche in einer geeigneten Kosmetikgrundlage zur Anwendung kommen.

Die Erfindung betrifft weiterhin neue makromolekulare, oberflächenaktive, quaternäre, N-substituierte Chitosanderivate.

Es ist bereits bekannt, in kosmetischen Mitteln, insbesondere für die Behandlung von Haaren, kationaktive Polymere, vorzugsweise Polymere, die quaternäre Ammoniumgruppen aufweisen, als Konditionierungsmittel einzusetzen. Auf Grund einer Wechselwirkung zwischen ihren Ammoniumgruppen und den anionischen Gruppen des Haares besitzen die kationaktiven Polymere eine große Affinität zur Keratinfaser.

Es wurde festgestellt, daß der Einsatz derartiger kationischer Polymere in solchen kosmetischen Mitteln zahlreiche Vorteile ergibt; die Entwirrung der Haare sowie deren Behandlung wird erleichtert, weiterhin werden dem Haar Sprungkraft und Glanz verliehen. Durch die Affinität zum Keratin neigen diese Polymere jedoch bei wiederholter Anwendung zur Ansammlung auf den Haaren, so daß diese schwerer werden, was im Endeffekt unerwünscht ist.

Weiterhin ergeben sich bei synthetischen Polymeren Probleme wegen der physiologischen Wirkung von eventuell vorhandenen Monomerspuren, die nur schwer aus dem Polymer entfernbar sind.

Man hat bereits versucht, diese vorerwähnten Nachteile dadurch zu beheben, daß wasserlösliche Salze des Chitosans, eines durch Deacetylierung von Chitin herstellbaren Polyglucosamins, in derartigen kosmetischen Mitteln Anwendung finden. In diesem Zusammenhang wird Bezug genommen auf die eigene europäische Patentschrift 0 002 506 sowie die eigene deutsche Patentschrift 26 27 419.

In gleicher Weise wie bei der Mehrzahl der kationaktiven Polymere mit quaternären Gruppierungen ergibt Chitosan häufig den Nachteil, daß es mit den üblicherweise in Haarbehandlungsmitteln, insbesondere in Shampoos, enthaltenen anionischen oberflächenaktiven Verbindungen nur wenig verträglich ist. Es ist daher notwendig, das Chitosan in separaten Behandlungen, nämlich vor und/oder nach der Shampoonierung zur Einwirkung zu bringen. Das Chitosan erweist sich weiterhin in neutralem und alkalischem Medium als praktisch unlöslich, so daß beispielsweise seine Anwendung in alkalischen Dauerwellmitteln oder Haarfärbemitteln nicht möglich ist.

Durch den Einsatz bestimmter wasserlöslicher kationaktiver Chitosanderivate gemäß den eigenen deutschen Offenlegungsschriften DE-OS 32 45 784, DE-OS 35 01 891 und DE-OS 35 02 833 anstelle von Chitosansalzen lassen sich die vorstehend erwähnten Nachteile größtenteils vermeiden. Es ist jedoch bei Verwendung dieser Chitosanderivate in Aerosolschaumpräparaten oder Emulsionen weiterhin erforderlich, diesen Mitteln oberflächenaktive Verbindungen, wie beispielsweise kationische Tenside, als Emulgatoren oder Schaumbildner zuzusetzen, da diese Chitosanderivate keine emulgierenden beziehungsweise schaumbildenden Eigenschaften besitzen. Durch den Zusatz von Emulgatoren wird jedoch die physiologische Verträglichkeit der Mittel beeinträchtigt, so daß es zu Reizungen der Haut, der Kopfhaut oder der Augen kommen kann.

Aufgabe der vorliegenden Erfindung war es daher, wasserlösliche Chitosanderivate für den Einsatz in kosmetischen Mitteln zur Verfügung zu stellen, die bei guter physiologischer Verträglichkeit sowohl festigende und konditionierende Eigenschaften als auch emulgierende und schaumbildende Eigenschaften besitzen.

Bei Fortführung der Untersuchungen mit Chitosan und den davon abgeleiteten Verbindungen wurde nunmehr gefunden, daß bestimmte wasserlösliche kationische Chitosanderivate sowohl die Eigenschaften eines Polymers als auch die eines Tensids besitzen.

Der Einsatz dieser neuen Chitosanderivate ermöglicht beispielsweise die Herstellung von Schaumfestigern mit hervorragenden Schaum- und Festigungseigenschaften ohne Verwendung von zusätzlichen Schaumbildnern oder Polymeren. Weiterhin sind diese neuen oberflächenaktiven Chitosanderivate bei Verwendung in Emulsionen in der Lage, gleichzeitig als Emulgator und Konditioniermittel zu wirken.

Im Gegensatz zu bisher verwendeten kosmetischen Mitteln auf der Basis von kationaktiven Tensiden oder Emulgatoren ist die physiologische Verträglichkeit des auf den neuen kationischen Chitosanderivaten basierenden Mittels sehr gut, da die Eindringtiefe der neuen Chitosanderivate in die Haut aufgrund ihrer Molekülgröße nur sehr gering ist.

Mit diesen neuen kationischen, oberflächenaktiven Chitosanderivaten läßt sich somit ein kosmetisches Mittel zur Behandlung von Haaren und der Haut herstellen, das sich durch überraschende vorteilhafte Eigenschaften auszeichnet und dadurch gekennzeichnet ist, daß es in einer wäßrigen oder wäßrigalkoholischen Zubereitung 0,05 bis 10 Gewichtsprozent einer makromolekularen, oberflächenaktiven, quaternären, vom Chitosan abgeleiteten, N-substituierten Verbindung der allgemeinen Formel (I)

HO $[C_6H_{11\text{-}x\text{-}y\text{-}z}NO_4(R^1)_x(R^2)_y(R^3)_z]_p$ H (I),

wobei
x jeden beliebigen Zahlenwert von 0,04 bis 0,4,
y jeden beliebigen Zahlenwert von 0,04 bis 0,92
und
z jeden beliebigen Zahlenwert von 0,04 bis 0,92
annehmen kann, mit der Bedingung, daß $(x+y+z) \leq 2$ ist,
p für eine ganze Zahl von 100 bis 100.000 steht,
$R^1$ den Rest

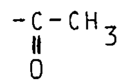

bedeutet,
$R^2$ den Rest

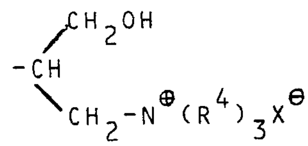

oder

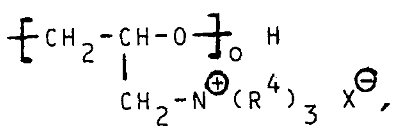

mit $R^4$ gleich $C_1$ bis $C_4$-Alkyl, X gleich Cl, Br, J, $CH_3SO_4$ und o gleich einer ganzen Zahl von 1 bis 5, darstellt und
$R^3$ den Rest

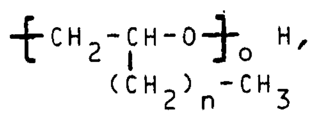

mit n gleich einer ganzen Zahl von 5 bis 20
und o gleich einer ganzen Zahl von 1 bis 5,
bedeutet, enthält.

Das erfindungsgemäße, ein quaternäres Chitosanderivat der Formel (I) enthaltende Mittel eignet sich ganz allgemein zur Behandlung der Haut und der Haare. Es kann zum Beispiel vorliegen als Aerosolschaum, Frisierlotion, Fönlotion, Mittel zur Festigung der Frisur, Waschlotion, Haarkur, Mittel gegen Kopfschuppen, Mittel zur permanenten Haarverformung, Mittel zur Färbung oder Entfärbung von Haaren, Mittel zum Auftragen vor oder nach der Haarfärbung und als kosmetisches Mittel zur Pflege, zum Schutz oder zur Reinigung der Haut. Beispiele für ein derartiges Mittel zur Pflege und zur Reinigung der Haut sind Gesichtswässer, Rasierwässer, Feuchthaltecremes, Coldcremes, Körperlotionen, Sonnenschutzmittel oder auch Make-up-Präparate.

Der Gehalt des erfindungsgemäßen kosmetischen Mittels an dem neuen Chitosanderivat der Formel (I) liegt zweckmäßig bei 0,05 bis 10 Gewichtsprozent, vorzugsweise 0,05 bis 3 Gewichtsprozent.

Das kosmetische Mittel gemäß der vorliegenden Erfindung kann zusätzlich zu dem neuen Chitosanderivat der Formel (I) zur Herstellung einer Kosmetikgrundlage alle diejenigen Bestandteile enthalten, die in Haar-und Hautbehandlungsmitteln überlicherweise eingesetzt werden, insbesondere anionische, kationische, amphotere, zwitterionische oder nichtionische oberflächenaktive Verbindungen (Tenside), Schaumsynergisten, Stabilisatoren, Sequestriermittel, Pigmente, Verdicker, Emulgatoren, Pufferstoffe, Konservierungsmittel, Farbstoffe, Parfümöle, bekannte kosmetische Polymere wie anionische, nichtionische, kationische oder amphotere Polymere, Naturstoffe, kosmetische Öle, Fettalkohole, Wachse, Schaumstabilisatoren, Wirkstoffe gegen Kopfschuppen, Reduktionsmittel und Treibgase.

Das erfindungsgemäße kosmetische Mittel weist in bevorzugter Weise einen pH-Wert von 2 bis 11 auf und kann in Form einer wäßrigen oder wäßrig-alkoholischen Zubereitung, zum Beispiel mit einem Alkohol mit 1 bis 4 Kohlenstoffatomen, als Lösung, als Creme, als Gel, als Dispersion oder als Emulsion, vorliegen. Ebenfalls ist es möglich, dieses Mittel mit Hilfe eines Zerstäubers beziehungsweise anderer geeigneter Sprühvorrichtungen zu versprühen oder im Gemisch mit üblichen unter Druck verflüssigten Treibgasen als Aerosolspray oder Aerosolschaum aus einem Druckbehälter zu entnehmen.

Wenn es sich bei dem erfindungsgemäßen kosmetischen Mittel um ein Mittel zur Festigung der Frisur, wie zum Beispiel einen Haarfestiger oder Haarspray, handelt, dann liegt es üblicherweise als wäßrige oder wäßrigalkoholische Lösung vor, die durch einen Gehalt an einem quaternären Chitosanderivat der vorstehend genannten Formel (I) gekennzeichnet ist. Hierbei kann das quaternäre Chitosanderivat der Formel (I) selbst als filmbildendes beziehungsweise festigendes Harz eingesetzt werden. Es können jedoch auch zusätzlich andere filmbildende, natürliche oder synthetische Polymere in dem erfindungsgemäßen Haarfestigungsmittel enthalten sein. Als natürliche Polymere kommen beispielsweise Schellack, Alginate, Gelatine, Pektine und Cellulosederivate in Betracht. Von den synthetischen Polymeren finden zum Beispiel Polyvinylpyrrolidon, Polyvinylacetat, Polyacrylverbindungen, wie Acrylsäure- oder Methacrylsäurepolymerisate, basische Polymerisate von Estern aus Acrylsäure oder Methacrylsäure mit Aminoalkoholen beziehungsweise die Salze oder Quaternisierungsprodukte dieser basischen Polymerisate, Polyacrylnitril sowie Co- oder Terpolymerisate aus derartigen Verbindungen, beispielsweise Polyvinylpyrrolidon-Vinylacetat, Verwendung.

Dieses Mittel weist dann insbesondere einen pH-Wert zwischen 4 und 8 auf. Ein solches Mittel zur Festigung der Frisur enthält üblicherweise die filmbildenden Polymeren in einer Gesamtmenge von etwa 0,05 bis 3,0 Gewichtsprozent. Enthält das Mittel neben dem quaternären Chitosanderivat der Formel (I) noch andere filmbildende Polymere, so reduziert sich der Gehalt an dem quaternären Chitosanderivat der Formel (I) entsprechend.

Als Alkohole kommen insbesondere die für kosmetische Zwecke üblicherweise verwendeten niederen Alkohole mit 1 bis 4 Kohlenstoffatomen, wie zum Beispiel Ethanol und Isopropanol, in Betracht.

Wenn das Mittel zur Festigung der Frisur in Form eines Schaumpräparates vorliegt, welches aus einem Druckbehälter entnommen wird, so enthält es in der Kosmetikgrundlage etwa 10 bis 60 Gewichtsprozent eines Treibmittels. Als Treibmittel können Chlorfluoralkane, wie zum Beispiel $CCl_3F$, $CCl_2F_2$, $C_2Cl_3F_3$, $(CCl_2F)_2$, $CHCl_2F$ und $(CClF_2)_2$, leichtflüchtige Kohlenwasserstoffe, wie zum Beispiel n-Butan und n-Propan, oder auch Dimethylether, Kohlendioxid, Distickstoffmonoxid, Stickstoff, Methylenchlorid und 1,1,1-Trichlorethan, Verwendung finden. In diesem Mittel dient das quaternäre, oberflächenaktive Chitosanderivat der Formel (I) gleichzeitig als Schaumbildner und als festigende Komponente.

Es können jedoch auch zusätzlich andere filmbildende, natürliche oder synthetische Polymere in dem erfindungsgemäßen Aerosolschaum enthalten sein.

Das erfindungsgemäße Mittel zur Festigung der Frisur kann weiterhin die für ein derartiges Mittel üblichen Zusätze wie beispielsweise Parfümöl, Bakterizide oder Fungizide, kämmbarkeitsverbessernde Substanzen und Modifiziermittel, wie zum Beispiel Siliconöl, oder Weichmacher, wie beispielsweise Isopropylmyristat, Phthalsäurediethylester und Diethylstearat, enthalten.

Das vorliegende Mittel zur Festigung der Frisur kann gegebenenfalls durch einen Gehalt an kosmetischen Farbstoffen das Haar gleichzeitig färben oder tönen. Derartige Präparate sind unter anderem als Farbfestiger oder Tönungsfestiger im Handel bekannt. Sie enthalten zusätzlich übliche, für Haarfestiger bekannte Farbstoffe, wie beispielsweise aromatische Nitrofarbstoffe (zum Beispiel 1,4-Diamino-2-nitrobenzol), Azofarbstoffe (zum Beispiel C.I. 14 805-Acid Brown 4), Anthrachinonfarbstoffe (zum Beispiel C. I. 61 105-Disperse Violet 4) und Triphenylmethanfarbstoffe (zum Beispiel C. I. 42 535-Basic Violet 1), wobei die Farbstoffe dieser Klassen je nach der Art ihrer Substituenten sauren, nichtionogenen oder basischen Charakter haben können. Ihre Gesamtkonzentration in diesen Präparaten beträgt üblicherweise etwa 0,01 bis 2,0 Gewichtsprozent.

Das erfindungsgemäße Mittel zur Festigung der Frisur weist bei gleich guter Festigung des Haares gegenüber üblichen Haarfestigern eine verbesserte Substantivität zum Haar, eine besonders gute Kämmbarkeit und einen guten Griff des Haares im nassen Zustand sowie einen besonders angenehmen Griff des Haares im getrockneten Zustand auf.

Weiterhin kann das hier beschriebene Mittel ein Haarwaschmittel darstellen. Es liegt dann in Form einer wäßrigen Lösung oder Emulsion vor und enthält neben dem neuen Chitosanderivat ein anionisches, kationisches, nichtionisches oder amphoteres Tensid.

In diesem Haarwaschmittel liegt die Konzentration des Tensides, bezogen auf das Gesamtgewicht des Mittels, im allgemeinen zwischen 3 und 50 Gewichtsprozent und vorzugsweise zwischen 3 und 25 Gewichtsprozent, wobei der pH-Wert im allgemeinen zwischen 3 und 9 und vorzugsweise zwischen 4 und 7 liegt.

Das erfindungsgemäße Mittel, das in Form eines Haarwaschmittels vorliegt, enthält im allgemeinen verschiedene Zusatzstoffe, insbesondere Parfüms, Konservierungsstoffe, Verdicker, Schaumstabilisatoren, Puffersubstanzen, kosmetische Harze, Pigmente und Farbstoffe.

Unter den Schaumstabilisatoren können die Fettamide und insbesondere die Mono- oder Diethanolamide von Copra-Fettsäuren, Lauryl- oder ölsäuremono- oder -diethanolamid, die zweckmäßigerweise in Mengen von 1 bis 10 und vorzugsweise 1 bis 3 Gewichtsprozent, bezogen auf das Gesamtgewicht des Mittels, Verwendung finden, angeführt werden.

Unter den Verdickern können insbesondere die Acrylpolymere und die Cellulosederivate, wie zum Beispiel Carboxymethylcellulose, Hydroxypropylmethylcellulose und Hydroxyethylcellulose, angeführt werden. Die Verdicker liegen im allgemeinen in einem Anteil von 0,1 bis 5 Gewichtsprozent vor.

Unter den Tensiden oder oberflächenaktiven Agenzien, die in Kombination mit den neuen quaternären Chitosanderivaten verwendet werden, können beispielsweise die folgenden genannt werden:

a) Die anionischen, oberflächenaktiven Agenzien, wie beispielsweise die Alkali-, Erdalkali-, Ammonium- oder Alkanolaminsalze von Alkansulfonaten, Alkylsulfaten und Alkylethersulfaten, die $C_{12}$-$C_{14}$-Alkyl-Sulfatnatriumsalze oder -Triethanolaminsalze, die Natrium- oder Triethanolaminsalze von Lauryl- oder Tetradecylethersulfaten, das Dinatriumsalz des Sulfosuccinhalbesters von Alkanolamiden, die Seifen und die Polyethercarbonsäuren;

b) die nichtionischen, oberflächenaktiven Agenzien, wie beispielsweise oxethylierte Fettalkohole mit 12 bis 18 Kohlenstoffatomen, zum Beispiel mit bis zu 40 Mol Ethylenoxid pro Mol Fettalkohol oxethylierter Lauryl-, Tetradecyl-, Cetyl-, Oleyl-, Palmityl- und Stearylalkohol, allein oder im Gemisch, die Fettalkohole von oxethyliertem Lanolin oder oxethyliertes Lanolin; Polyglycerinether von gesättigten oder ungesättigten Fettalkoholen und Alkylphenolen mit 8 bis 30 Kohlenstoffatomen im Alkylrest und 1 bis 10 Glyceryleinheiten im Molekül, sowie Fettsäurealkanolamide;

c) die kationischen, oberflächenaktiven Agenzien, wie beispielsweise das Dilauryldimethylammoniumchlorid, die Chloride oder Bromide von Alkyldimethylbenzylammonium, beispielsweise Cetyltrimethylammoniumchlorid oder -bromid, Tetradecyltrimethylammoniumchlorid oder -bromid, die Alkyldimethylhydroxyethylammoniumchloride oder -bromide, Alkylpyridiniumsalze, beispielsweise Cetylpyridiniumchlorid, die Alkylamidethyltrimethylammoniumethersulfate, Imidazolinderivate, Verbindungen mit kationischem Charakter wie Aminoxide, beispielsweise Alkyldimethylaminoxide oder Alkylaminoethyldimethylaminoxide;

d) die amphoteren oder zwitterionischen, oberflächenaktiven Agenzien wie beispielsweise die Carboxylderivate von Imidazol, die N-Alkylbetaine, die N-Alkylsulfobetaine, die N-Alkylaminobetaine, die N-Alkylaminopropionate, die Alkyldimethylammoniumacetate sowie die $C_{12}$-$C_{18}$-Alkyldimethyl-carboxymethylammoniumsalze

Das erfindungsgemäße kosmetische Mittel kann auch eine Creme oder Lotion zur Verwendung als Haarkur oder Hautpflegemittel darstellen. Es liegt dann meist in Form einer Öl-in-Wasser- oder Wasser-in-Öl-Emulsion oder Suspension vor und kann zusätzlich zu dem neuen Chitosanderivat der Formel (I) kationische, nichtionogene, amphotere oder anionische Emulgatoren sowie als Bestandteil der Ölphase zum Beispiel Fettalkohole, Fettsäureester oder -amide, weiterhin Parfümöle, Vaseline, Wollfettalkohol oder feste oder flüssige Paraffine enthalten.

Wenn das hier beschriebene Mittel eine Emulsion darstellt, so wird das quaternäre, oberflächenaktive Chitosanderivat in bevorzugter Weise als Emulgator eingesetzt und dient wegen seiner kationischen Komponente gleichzeitig der Konditionierung des Haares. Es können jedoch auch übliche als Emulgator dienende Tenside zusätzlich als Co-Emulgatoren zugesetzt werden.

Wenn das erfindungsgemäße Mittel ein Haartönungs- oder Haarfärbemittel darstellt, so liegt es bevorzugt in Form einer Creme oder Lotion vor und enthält zusätzlich übliche Haarfarbstoffe aus der Gruppe der aromatischen Nitrofarbstoffe, Azofarbstoffe, Anthrachinonfarbstoffe, Triphenylmethanfarbstoffe oder auch Oxidationsfarbstoffe, beispielsweise aus der Gruppe der aromatischen Diamide beziehungsweise Aminophenole. Weiterhin kann dieses Mittel gegebenenfalls Alkalisierungsmittel, Antioxidantien sowie weitere für ein derartiges Mittel übliche kosmetische Zusätze und Hilfsstoffe enthalten.

Das vorliegende Mittel kann auch ein Dauerverformungsmittel oder Fixiermittel für die Haare darstellen.

Es enthält dann zusätzlich zu den genannten Chitosanderivaten der Formel (I) ein Reduktionsmittel, wie zum Beispiel Thioglykolsäure, Thiomilchsäure und Ammoniumsulfit, beziehungsweise ein Oxidationsmittel, wie beispielsweise Wasserstoffperoxid oder Natriumbromat, sowie gegebenenfalls Alkalisierungsagenzien beziehungsweise Peroxidstabilisatoren, zum Beispiel Phosphorsäure, und andere kosmetische Hilfsstoffe und Zusatzstoffe, wie beispielsweise Parfümöle, Riechstoffe, Pflegestoffe und Farbstoffe.

Wie bereits erwähnt wurde, kann das erfindungsgemäße kosmetische Mittel auch zur Behandlung der Haut verwendet werden.

In der Tat erleichtert dieses kosmetische Mittel die Befeuchtung der Haut und verhindert das Austrocknen. Dieses Mittel verleiht der Haut weiterhin eine hervorragende Weichheit im Griff.

Das erfindungsgemäße kosmetische Mittel liegt hierzu vorzugsweise in Form eines Schaumes, aber auch in Form einer Creme, eines Gels, einer Emulsion oder einer wäßrigen oder wäßrig-alkoholischen Lösung vor, wobei das Chitosanderivat der Formel (I) in einer Konzentration von 0,1 bis 10 Gewichtsprozent, vorzugsweise 0,2 bis 6 Gewichtsprozent, enthalten ist.

Die im allgemeinen in einer derartigen Kosmetikzubereitung enthaltenen Hilfsstoffe sind beispielsweise Duftstoffe, Farbstoffe, Konservierungsmittel, Verdickungsmittel, Sequestriermittel, Emulgiermittel, Sonnenschutzfilter und ähnliche.

Diese Zubereitung für die Hautpflege liegt insbesondere in Form eines Schaumpräparates, einer Creme oder Lotion zur Pflege der Hände oder des Gesichts oder in Form einer Sonnenschutzcreme, einer gefärbten Creme, eines Abschminkmilchproduktes, eines Schaum- oder Duschbad-Präparates oder auch in Form einer Deodorierzubereitung vor.

Diese Zubereitung wird unter Anwendung klassischer Verfahrensweisen hergestellt. Beispielsweise kann man zur Bildung einer Creme eine wäßrige Phase, die das erfindungsgemäße Chitosanderivat der Formel (I) und gegebenenfalls andere Bestandteile oder Hilfsstoffe gelöst enthält, und eine ölige Phase emulgieren. Für die ölige Phase kann man verschiedenartige Verbindungen, beispielsweise Paraffinöl, Vaselinöl, Süßmandelöl, Avocadoöl, Olivenöl, Fettsäureester, wie zum Beispiel Glycerylmonostearat, Ethylpalmitat und Isopropylpalmitat, oder Alkylmyristate, wie zum Beispiel Propylmyristat, Butylmyristat, oder Fettalkohole und Wachse, beispielsweise Wollwachs oder Bienenwachs, verwenden.

Das Chitosanderivat der Formel (I) kann in dieser Kosmetikzubereitung für die Hautpflege entweder als Hilfsstoff oder als Hauptwirkstoff enthalten sein.

Die in dem erfindungsgemäßen kosmetischen Mittel enthaltenen neuen quaternären, oberflächenaktiven Chitosanderivate der Formel (I) leiten sich vom Chitosan ab, einem Material, das durch Deacetylierung von Chitin, einem natürlich vorkommenden Acetylglucosamin, erhalten wird.

Das Chitosan ist im neutralen und alkalischen Medium unlöslich, es bildet jedoch aufgrund seiner chemischen Natur im sauren Medium mit bestimmten organischen und anorganischen Säuren lösliche Salze. Aufgrund seiner chemischen Struktur sowie seiner Eigenschaften eignet sich Chitosan beispielsweise für die Anwendung in Pharmazeutika und Kosmetika, in der Landwirtschaft, oder als Ionenaustauscher. Die kommerzielle Nutzung des Chitosans ist jedoch, nicht zuletzt wegen seiner Unlöslichkeit im wäßrigen Medium, auf den Einsatz als Flockungsmittel in der Abwasseraufbereitung beschränkt geblieben.

Um die Anwendungsmöglichkeiten des Chitosans zu erweitern, wurden bereits mehrere wasserlösliche kationaktive Chitosanderivate hergestellt (siehe hierzu unsere eigenen deutschen Patentanmeldungen DE-OS 32 45 784, DE-OS 35 01 891 und DE OS 35 02 833). Diese kationaktiven Chitosanderivate sollen insbesondere zur Festigung oder Konditionierung der Haare verwendbar sein.

Es wurde nunmehr gefunden, daß durch gleichzeitige Umsetzung von Chitosan mit einem Glycidyltrialkylammoniumhalogenid (Oxyranmethanammonium-N,N,N-trialkylhalogenid) der Formel (II)

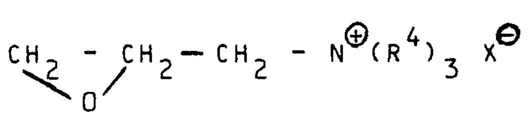

(II)

($R^4$ = $C_1$ bis $C_4$ - Alkyl, X = Cl, Br, J, $CH_3SO_4$)

und einem Epoxid mit langer Alkylkette in einfacher Weise neue oberflächenaktive kationische Chitosanderivate hergestellt werden können. Diese neuen, in Wasser sowie in Wasser/Alkohol-Mischungen löslichen, Chitosanderivate besitzen sowohl Polymer- als auch Tensideigenschaften und sind deshalb für die Verwendung in einer Vielzahl von kosmetischen Mitteln, insbesondere Emulsionen und Schaumpräparaten, geeignet.

Gegenstand der vorliegenden Erfindung sind daher weiterhin makromolekulare, oberflächenaktive,

quaternäre, vom Chitosan abgeleitete, N-substituierte Verbindungen der allgemeinen Formel (I)

$HO\ [C_6H_{11-x-y-z}\ NO_4\ (R^1)_x\ (R^2)_y\ (R^3)_z]_p\ H$ (I),

wobei
x jeden beliebigen Zahlenwert von 0,04 bis 0,4,
y jeden beliebigen Zahlenwert von 0,04 bis 0,92
und
z jeden beliebigen Zahlenwert von 0,04 bis 0,92
annehmen kann, mit der Bedingung, daß $(x+y+z) \leq 2$ ist,
p für eine ganze Zahl von 100 bis 100.000 steht,
$R^1$ den Rest

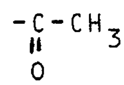

bedeutet,
$R^2$ den Rest

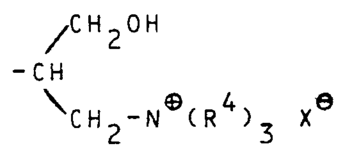

oder

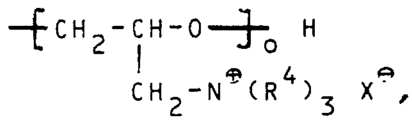

mit $R^4$ gleich $C_1$ bis $C_4$-Alkyl, X gleich Cl, Br, J, $CH_3SO_4$ und o gleich eine ganze Zahl von 1 bis 5, darstellt und
$R^3$ den Rest

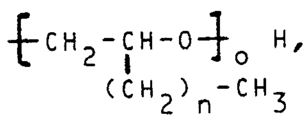

mit n gleich einer ganzen Zahl von 5 bis 20
und o gleich einer ganzen Zahl von 1 bis 5;
bedeutet.

Die neuen Chitosanderivate werden in Analogie zu dem in unserer eigenen deutschen Offenlegungsschrift 32 45 784 beschriebenen einstufigen Verfahren erhalten, indem man Chitosan (zu 60 bis 96 Prozent deacetyliertes Chitin) in einem geeigneten Lösungsmittel beziehungsweise Lösungsmittelgemisch bei Temperaturen zwischen 20 und 120 Grad Celsius, vorzugsweise zwischen 80 und 100 Grad Celsius, unter Druck im Autoklaven, in einem geeigneten Verhältnis mit einem langkettigen Epoxid sowie einem Glycidyltrialkylammoniumhalogenid über einen Zeitraum von 5 bis 100 Stunden, vorzugsweise 6 bis 24 Stunden, umsetzt.

Geeignete langkettige Epoxide sind insbesondere alpha-Epoxide mit acht (Octen-1-oxid) bis sechzehn (Hexadecen-1-oxid) Kohlenstoffatomen. Geeignete Glycidyltrialkylammoniumhalogenide sind solche, die $C_1$ bis $C_4$-Alkylgruppen enthalten, wie zum Beispiel Glycidyltrimethylammoniumchlorid und Glycidyltriethylammoniumchlorid.

Gemäß einer besonders vorteilhaften Ausführungsform dieses Herstellungsverfahrens wird als Ausgangsmaterial ein durch Umfällen und Tiefgefrieren strukturell modifiziertes Chitosan verwendet. Durch Verwendung dieses Chitosans verläuft die Reaktion in besonders vorteilhafter Weise und mit besonders hoher Ausbeute.

In bevorzugter Weise wird die Reaktion in einem Wasser/Alkohol-Gemisch, beispielsweise einem Wasser/Ethanol-Gemisch, durchgeführt.

Nach beendeter Reaktion trennt man eventuell vorhandene unlösliche Bestandteile aus den Lösungen des Chitosanderivates durch Filtration oder Zentrifugation ab, neutralisiert gegebenenfalls, engt die Reaktionsmischung im Vakuum ein und fällt die Chitosanderivate unmittelbar oder nach der Dialyse in Aceton, Alkoholen oder anderen geeigneten Lösungsmitteln aus.

Die nachfolgenden Beispiele sollen den Gegenstand der Erfindung näher erläutern, ohne diesen hierauf zu beschränken.

**Herstellungsbeispiele**

**Beispiel 1 Umsetzung von Chitosan mit Octen-1-oxid und Glycidyltrimethylammoniumchlcrid**

50 g (0,31 mol) niedermolekulares Chitosan mit einer Grenzviskositätszahl (Eta) von 140 ml/g und einem Deacetylierungsgrad von 86 Prozent werden in der äquimolaren Menge Salzsäure gelöst und anschließend durch Einstellen eines pH-Wertes von 8 mit Natronlauge ausgefällt. Das ausgefällte und abgesaugte, wasserhaltige Chitosan wird gewogen und mit der der enthaltenen Wassermenge entsprechenden Menge Ethanol versetzt.

Die so erhaltene Aufschlämmung von Chitosan in Ethanol/Wasser (1:1) wird zusammen mit 159 g (1,24 mol) Octen-1-oxid sowie 79 g (0,34 mol) einer 65-prozentigen wäßrigen Lösung von Glycidyltrimethylammoniumchlorid 6 Stunden lang bei 100 Grad Celsius gerührt. Die dunkelbraune, fast klare Reaktionsmischung wird sodann filtriert und anschließend im Rotationsverdampfer auf ungefähr die Hälfte eingeengt.

Durch Eintropfen der Reaktionsmischung in 5 bis 8 Liter Aceton wird das Chitosan ausgefällt. Es wird abgesaugt und sodann im Vakuum getrocknet.

Die Ausbeute beträgt 72,3 g.

Kenndaten:

Grenzviskositätszahl (Eta): 74 ml/g
Titrierbarer Stickstoff: 3,76 mmol/g
Titrierbarer quaternärer Stickstoff: 1,60 mmol/g
Substitutionsgrad kationisch: 0,42
Substitutionsgrad Octyl: 0,31

**Beispiel 2 Umsetzung von Chitosan mit Hexadecen-1-oxid und Glycidyltrimethylammoniumchlorid**

50 g (0,31 mol) niedermolekulares Chitosan mit den gleichen Kenndaten wie in Beispiel 1 werden in der in Beispiel 1 beschriebenen Weise umgefällt und mit der entsprechenden Menge Ethanol versetzt.

Anschließend wird die so erhaltene Mischung zusammen mit 288 g (1,24 mol) Hexadecen-1-oxid sowie 79 g (0,34 mol) einer 65-prozentigen wäßrigen Lösung von Glycidyltrimethylammoniumchlorid im Autoklaven bei 100 Grad Celsius 24 Stunden lang umgesetzt.

Die Reaktionsmischung wird anschließend filtriert, sodann wird das Filtrat eingeengt und das Chitosanderivat in Aceton ausgefällt. Das erhaltene Reaktionsprodukt wird abgesaugt, bei 50 Grad Celsius im Vakuum getrocknet und anschließend gemahlen.

Die Ausbeute beträgt 84,3 g.

Kenndaten:

Grenzviskositätszahl (Eta): 65 ml/g
Titrierbarer Stickstoff: 3,66 mmol/g
Titrierbarer quaternärer Stickstoff: 1,95 mmol/g
Substitutionsgrad kationisch: 0,53
Substitutionsgrad Hexadecyl: 0,13

**Beispiel 3 Umsetzung von Chitosan mit Tetradecen-1-oxid und Glycidyltrimethylammoniumchlorid**

50 g (0,31 mol) Chitosan mit den gleichen Kennzahlen wie in Beispiel 1 werden in der in Beispiel 1 beschriebenen Weise vorbehandelt und anschließend zusammen mit 263 g (1,24 mol) Tetradecen-1-oxid

und 79 g (0,34 mol) einer 65-prozentigen wäßrigen Lösung von Glycidyltrimethylammoniumchlorid im Autoklaven bei 100 Grad Celsius 6 Stunden lang gerührt.

Nach Beendigung der Reaktion wird das Chitosanderivat durch Ausfällen in Aceton isoliert und sodann in der in Beispiel 1 und 2 beschriebenen Weise gereinigt und getrocknet.

Die Ausbeute beträgt 72,1 g.

Kenndaten:

Grenzviskositätszahl (Eta): 97 ml/g
Titrierbarer Stickstoff: 4,10 mmol/g
Titrierbarer quaternärer Stickstoff: 2,11 mmol/g
Substitutionsgrad kationisch: 0,51
Substitutionsgrad Tetradecyl: 0,02

**Beispiel 4 Umsetzung von hochmolekularem Chitosan mit Octen-1-oxid und Glycidyltrimethylammoniumchlorid**

25 g (0,155 mol) eines hochmolekularen Chitosans mit einer Grenzviskositätszahl (Eta) von 1600 ml/g und einem Deacetylierungsgrad von 76 Prozent werden analog Beispiel 1 umgefällt und mit der der enthaltenen Menge Wasser entsprechenden Menge Ethanol versetzt.

Die so erhaltene Chitosan/Wasser/Ethanol-Mischung wird anschließend zusammen mit 79,5 g (0,62 mol) Octen-1-oxid sowie 53,5 g (0,23 mol) einer 65-prozentigen wäßrigen Lösung von Glycidyltrialkylammoniumchlorid 6 Stunden lang im Autoklaven bei 100 Grad Celsius zur Reaktion gebracht.

Nach Beendigung der Reaktion wird das Reaktionsprodukt in Aceton ausgefällt, abgesaugt, zweimal mit Aceton gewaschen und sodann bei 50 Grad Celsius im Vakuum getrocknet.

Die Ausbeute beträgt 37,5 g.

Kenndaten:

Grenzviskositätszahl (Eta): 406 ml/g
Titrierbarer Stickstoff: 3,36 mmol/g
Titrierbarer quaternärer Stickstoff: 1,99 mmol/g
Substitutionsgrad kationisch: 0,59
Substitutionsgrad Octyl: 0,36

Die Messung der Grenzviskositätszahl (Eta) erfolgte in einer wäßrigen Lösung von 0,2 mol/l Essigsäure und 0,1 mol/l Natriumacetat (Chitosan) beziehungsweise in einer wäßrigen Lösung von 0,2 mol/l Essigsäure und 0,1 mol/l Natriumchlorid (Chitosanderivate) bei 25 Grad Celsius unter Verwendung eines DIN-Ubbelohde-Kapillarviskosimeters.

Die Bestimmung des Substitutionsgrades wurde wie folgt durchgeführt:

Zunächst wurde durch eine nichtwäßrige Titration mit Perchlorsäure die Molmenge an titrierbarem Stickstoff ($N_t$) ermittelt. Das mittlere Molekulargewicht ($\overline{M}$) wurde daraus wie folgt berechnet:

$$\overline{M}\,[g/mol] = \frac{1000}{N_t\,[mmol/g]}$$

Weiterhin gilt: $\overline{M} = 161 + (x \cdot 42) + (y \cdot 152) + (z \cdot m)$

- Molekulargewicht einer Chitosaneinheit = 161 g/mol
- Molekulargewicht einer quaternären Gruppe $R^2$ aus der Formel (I) ($R^4 = CH_3$;X=Cl) = 152 g/mol
- Molekulargewicht einer $CO\text{-}CH_2$-Gruppe = 42 g/mol
- Molekulargewicht einer Alkylgruppe $R^3$ aus Formel (I)[$R^3$(Octyl) = 129; $R^3$(Tetradecyl) = 213; $R^3$(Hexadecyl) = 241 ] = m

x = Substitutionsgrad des verwendeten Chitosans (0,04 bis 0,4)
y = Substitutionsgrad kationische Gruppen $R^2$
z = Substitutionsgrad Alkylgruppen $R^3$

Für die Berechnung des Substitutionsgrades mit den kationischen Gruppen $R^2$ wurde zunächst der titrierbare quaternäre Stickstoff ($N_{tq}$) durch eine Kolloid-Titration mit Polyvinylsulfat-Kaliumsalz bestimmt und

anschließend mit Hilfe der Gleichung

$$y = \frac{N_{tq} \cdot \bar{M}}{1000}$$

der Substitutionsgrad berechnet.

Der Substitutionsgrad mit Alkylgruppen wurde wie folgt berechnet:

$$z = \frac{\bar{M} - 161 - (152 \cdot y) - (42 \cdot x)}{m}$$

**Beispiel 5**

Untersuchung der oberflächenaktiven Eigenschaften der Chitosanderivate der Beispiele 1 bis 4

Als Vergleichssubstanz dient ein kationisches Chitosanderivat gemäß Beispiel 2 der DE-OS 32 45 784, welches als nichtionische Komponente Glycidol enthält. Die Kenndaten dieser Verbindung sind:
Grenzviskosität (Eta): 65 ml/g
Titrierbarer Stickstoff 3,07 mmol/g
Substitutionsgrad kationisch: 0,22
Substitutionsgrad Glycidol: 1,7

a) Schaumbildungseigenschaften einer 0,5-prozentigen wäßrigen Lösung (pH = 5)

100 ml einer 0,5-prozentigen wäßrigen Lösung des zu untersuchenden Chitosanderivates wurden in einem mit einem Schliffstopfen verschlossenen 250-ml-Meßzylinder durch zehnmaliges Drehen des Meßzylinders um 180 Grad geschüttelt. Anschließend wurde die Schaumhöhe (in ml) abgelesen. Nach Ablauf von 5 Minuten wurde die Schaumhöhe erneut gemessen.

| untersuchte Chitosanverbindung | Schaumhöhe [ml] | |
|---|---|---|
| | sofort | nach 5 Minuten |
| gemäß Beispiel 1 | 165 | 165 |
| gemäß Beispiel 2 | 124 | 120 |
| gemäß Beispiel 3 | 138 | 134 |
| gemäß DE-OS 32 45 784 | 103 | 100 |

b) Aerosol-Schaumbildung einer 0,5-prozentigen wäßrigalkoholischen Lösung (70:30)

95 g einer 0,5-prozentigen wäßrig-alkoholischen Lösung (70:30) der zu untersuchenden Chitosanverbindung wurden zusammen mit 5 g Propan/Butan (70:30) in einer Druckgaspackung abgefüllt. Anschließend wurde das Schaumvolumen einer 10 g schweren Probe ermittelt.

Die Ergebnisse dieser Untersuchung sind in der nachstehenden Tabelle zusammengefaßt.

| Chitosanderivat gemäß | Schaumvolumen [ml] | Schaumqualität | Schaumstabilität |
|---|---|---|---|
| Beispiel 1 | 150 | feinporig, primär sahnig | Schaum bleibt länger als 10 Minuten stabil |
| Beispiel 2 | 100 | feinporig, primär sahnig | Schaum bleibt länger als 10 Minuten stabil |
| Beispiel 3 | 120 | feinporig, primär sahnig | Schaum bleibt länger als 10 Minuten stabil |
| Beispiel 4 | 110 | feinporig, primär kompakt | Schaum bleibt länger als 10 Minuten stabil |
| DE-OS 32 45 784 | 15 | wäßrig, keine Schaumbildung | instabil |

c) Emulgiereigenschaften

Es wurde versucht, eine Emulsion der folgenden Zusammensetzung herzustellen:

| | |
|---|---|
| 4 g | Fettalkohol |
| 1 g | Chitosanderivat |
| 95 g | Wasser |
| 100 g | |

Ergebnis:

| Chitosanderivat gemäß | Beispiel 1 | DE-OS 3245784 |
|---|---|---|
| Emulsionseigenschaften | homogen, bei 40 Grad Celsius lagerstabil | inhomogen, nicht lagerstabil |

**Beispiele für kosmetische Mittel**

**Beispiel 6 Schaumfestiger**

| | |
|---|---|
| 1,0 g | quaternäres, oberflächenaktives Chitosanderivat nach Beispiel 1 (Eta = 74 ml/g; Substitutionsgrad: kationisch 0,42; Octyl 0,31) |
| 30,0 g | Ethanol |
| 5,0 g | Treibgas (Propan/Butan 70/30) |
| 0,2 g | Parfümöl |
| 63,8 g | Wasser |
| 100,0 g | |

Eine etwa golfballgroße Menge des feinporigen Schaumes wird auf gewaschenes, handtuchtrockenes Haar verteilt.

Anschließend werden die Haare in üblicher Weise frisiert und getrocknet.

Bei guter Festigungswirkung zeigt das Haar im Vergleich zu einem Schaumfestiger mit handelsüblichen synthetischen Polymeren einen angenehmeren und weicheren Griff.

**Beispiel 7 Haarkurmittel**

| | |
|---|---|
| 1,0 g | quaternäres, oberflächenaktives Chitosanderivat nach Beispiel 1 (Eta = 74 ml/g; Substitutionsgrad: kationisch 0,42; Octyl 0,31) |
| 4,0 g | Cetylstearylalkohol |
| 0,2 g | Parfüm |
| 94,8 g | Wasser |
| 100,0 g | |

35 g dieses Kurmittels werden im gewaschenen Haar verteilt und nach einer Einwirkungszeit von 3 bis 5 Minuten mit Wasser wieder ausgespült.

Als Ergebnis werden ein ausgezeichneter Griff und Glanz sowie eine sehr gute Kämmbarkeit des Haares erhalten.

**Beispiel 8 Schaumdauerwelle**

| | |
|---|---|
| 1,2 g | quaternäres, oberflächenaktives Chitosanderivat nach Beispiel 2 (Eta = 67 ml/g; Substitutionsgrad: kationische 0,53; Hexadecyl 0,13) |
| 10,0 g | Thioglykolsäure |
| 8,0 g | Ammoniak, 25-prozentige wäßrige Lösung |
| 6,1 g | Ammoniumhydrogencarbonat |
| 74,7 g | Wasser |
| 100,0 g | |

Die Wirkstofflösung wird mit Treibgas F 12/114 (1:1) im Verhältnis 85 : 15 abgefüllt.

Zur Anwendung trägt man diesen Aerosolschaum auf das gewickelte, handtuchtrockene Haar gleichmäßig auf und läßt ihn etwa 20 Minuten einwirken. Danach wird das Haar mit Wasser ausgespült und in bekannter Weise oxidativ behandelt.

Es wird ein gutes Wellenergebnis erhalten, die Haare fühlen sich natürlich und weich an.

**Beispiel 9 Haarfestiger**

| | |
|---|---|
| 1,3 g | quaternäres, oberflächenaktives Chitosanderivat nach Beispiel 3 (Eta = 97 ml/g; Substitutionsgrad: kationisch 0,51; Tetradecyl 0,02) |
| 35,0 g | Isopropanol |
| 0,4 g | Ameisensäure, 10-prozentige wäßrige Lösung |
| 0,2 g | Parfümöl |
| 63,1 g | Wasser |
| 100,0 g | |

20 ml dieser Lösung werden auf dem gewaschenen, handtuchtrockenen Haar verteilt. Sodann wird das Haar in üblicher Weise frisiert und getrocknet.

Bei guter Festigungswirkung zeigt das Haar im Vergleich zu einem Haarfestiger auf der Basis von Chitosan/Ameisensäure einen angenehmeren und weicheren Griff.

**Beispiel 10 Rasierschaum**

| | |
|---|---|
| 1,0 g | quaternäres, oberflächenaktives Chitosanderivat nach Beispiel 1 (Eta = 74 ml/g; Substitutionsgrad: kationisch 0,42; Octyl 0,31) |
| 40,0 g | Ethanol |
| 0,5 g | Parfüm |
| 0,2 g | Allantoin |
| 0,1 g | Konservierungsmittel |
| 58,2 g | Wasser |
| 100,0 g | |

Der Schaum verleiht der Haut nach der Rasur ein angenehmes, glattes Gefühl und desinfiziert gleichzeitig.

**Beispiel 11 Anionisches Haarwaschmittel**

| | |
|---|---|
| 1,00 g | quaternäres, oberflächenaktives Chitosanderivat nach Beispiel 4 (Eta = 406 ml/g; Substitutionsgrad: kationisch 0,59; Octyl 0,36) |
| 40,00 g | Laurylalkoholdiglykolethersulfat-Natriumsalz, 28-prozentige wäßrige Lösung |
| 4,00 g | Natriumchlorid |
| 0,10 g | Formaldehyd |
| 0,05 g | Farbstoff |
| 54,85 g | Wasser |
| 100,0 g | |

Es wird ein klares Shampoo erhalten. Das damit gewaschene Haar ist hinsichtlich Griff, Glanz und Kämmbarkeit ausgezeichnet konditioniert.

Insbesondere sind die hervorragenden Schaumeigenschaften des Shampoos durch Zusatz des Chitosanderivates hervorzuheben.

**Beispiel 12 Haarwasser in Schaumform**

| | |
|---|---|
| 0,5 g | quaternäres, oberflächenaktives Chitosanderivat nach Beispiel 3 (Eta = 97 ml/g; Substitutionsgrad: kationisch 0,51; Tetradecyl 0,02) |
| 40,0 g | Ethanol |
| 0,3 g | Parfümöl |
| 59,2 g | Wasser |
| 100,0 g | |

Das Haarwasser verleiht dem Haar nach dem Einmassieren in die Kopfhaut Frische und Glanz.

**Beispiel 13 Haarkurmittel**

| | |
|---|---|
| 1,0 g | quaternäres, oberflächenaktives Chitosanderivat nach Beispiel 1 (Eta = 74 ml/g; Substitutionsgrad: kationisch 0,42; Octyl 0,31) |
| 4,0 g | Cetylstearylalkohol |
| 1,0 g | Kokos(pentaethoxy)methylammoniumchlorid |
| 0,2 g | Parfüm |
| 93,8 g | Wasser |
| 100,0 g | |

35 g des obigen Kurmittels werden im gewaschenen Haar verteilt und nach einer Einwirkungszeit von 3 bis 5 Minuten mit Wasser wieder ausgespült.

Als Ergebnis werden ein ausgezeichneter Griff und Glanz sowie eine hervorragende Kämmbarkeit des Haares erhalten.

**Beispiel 14 Schaumfestiger**

| | |
|---|---|
| 1,0 g | quaternäres, oberflächenaktives Chitosanderivat nach Beispiel 1 (Eta = 74 ml/g; Substitutionsgrad: kationisch 0,42; Octyl 0,31) |
| 30,0 g | Ethanol |
| 5,0 g | Treibgas (Propan/Butan 70:30) |
| 2,0 g | Polyvinylprrolidon-Vinylacetat-Copolymer(im Verhältnis 30 : 70) |
| 0,2 g | Parfümöl |
| 61,8 g | Wasser |
| 100,0 g | |

Eine etwa golfballgroße Menge des feinporigen Schaumes wird auf gewaschenes, handtuchtrockenes Haar verteilt. Anschließend werden die Haare in üblicher Weise frisiert.

Bei starker Festigungswirkung zeigt das Haar einen angenehmen und weichen Griff.

**Beispiel 15 Roll-on Deo**

| | |
|---|---|
| 1,0 g | quaternäres, oberflächenaktives Chitosanderivat nach Beispiel 4 (Eta = 406 ml/g; Substitutionsgrad: kationisch 0,59; Octyl 0,36) |
| 40,0 g | Ethanol |
| 8,0 g | basisches Aluminiumchlorid (Aluminiumhydroxychlorid) |
| 1,0 g | Parfümöl |
| 0,2 g | Allantoin |
| 0,1 g | Konservierungsmittel |
| 49,7 g | Wasser |
| 100,0 g | |

Alle Prozentangaben stellen Gewichtsprozent dar.

## Patentansprüche

1. Kosmetisches Mittel zur Behandlung von Haaren und der Haut, dadurch gekennzeichnet, daß es in einer wäßrigen oder wäßrigalkoholischen Zubereitung 0,05 bis 10 Gewichtsprozent einer makromolekularen, oberflächenaktiven, quaternären, vom Chitosan abgeleiteten, N-substituierten Verbindung der allgemeinen Formel (I)

$$HO\,[C_6H_{11-x-y-z}\,NO_4\,(R^1)_x\,(R^2)_y\,(R^3)_z]_p\,H \qquad (I),$$

wobei
x jeden beliebigen Zahlenwert von 0,04 bis 0,4,
y jeden beliebigen Zahlenwert von 0,04 bis 0,92
und
z jeden beliebigen Zahlenwert von 0,04 bis 0,92

annehmen kann, mit der Bedingung, daß $(x + y + z) \leq 2$ ist,

p für eine ganze Zahl von 100 bis 100.000 steht,

$R^1$ den Rest

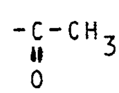

bedeutet,

$R^2$ den Rest

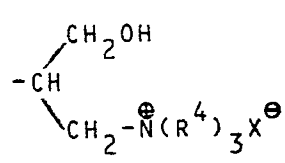

oder

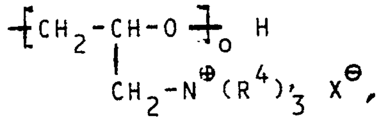

mit $R^4$ gleich $C_1$ bis $C_4$-Alkyl, X gleich Cl, Br, J, $CH_3SO_4$ und o gleich einer ganzen Zahl von 1 bis 5, darstellt und

$R^3$ den Rest

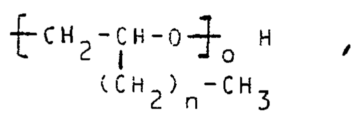

mit n gleich einer ganzen Zahl von 5 bis 20
und o gleich einer ganzen Zahl von 1 bis 5,

bedeutet, enthält.

**2.** Mittel nach Anspruch 1, dadurch gekennzeichnet, daß es als Lösung, Creme, Gel, Dispersion oder Emulsion vorliegt.

**3.** Mittel nach Anspruch 1 oder 2, dadurch gekennzeichnet, daß der pH-Wert 2 bis 11 beträgt.

**4.** Mittel nach einem der Ansprüche 1 bis 3, dadurch gekennzeichnet, daß es zusätzlich ein bekanntes filmbildendes synthetisches oder natürliches kosmetisches Polymer enthält.

**5.** Mittel nach einem der Ansprüche 1 bis 4, dadurch gekennzeichnet, daß es zusätzlich mindestens einen kosmetischen Farbstoff in einer Konzentration von 0,01 bis 2,0 Gewichtsprozent enthält und in Form eines Farbfestigers oder Tönungsfestigers vorliegt.

**6.** Mittel nach einem der Ansprüche 1 bis 5, dadurch gekennzeichnet, daß es als Kosmetikgrundlage eine wäßrige oder wäßrig-alkoholische Zubereitung enthält, die mit einem unter Druck verflüssigten Treibmittel vermischt ist, in einem Druckbehälter abgefüllt ist und in Form eines Aerosolschaums vorliegt.

**7.** Mittel nach einem der Ansprüche 1 bis 6, dadurch gekennzeichnet, daß es in Form eines Haarwaschmittels vorliegt und zusätzlich mindestens ein kationisches, nichtionisches, amphoteres oder anionisches Tensid enthält.

**8.** Mittel nach Anspruch 7, dadurch gekennzeichnet, daß es das Tensid in einer Konzentration zwischen 3 und 50 Gewichtsprozent enthält und einen pH-Wert zwischen 3 und 9 aufweist.

**9.** Makromolekulare, oberflächenaktive, quaternäre, vom Chitosan abgeleitete, N-substituierte Verbindung der allgemeinen Formel (I)

$$HO\,[C_6H_{11\text{-}x\text{-}y\text{-}z}\,NO_4\,(R^1)_x\,(R^2)_y\,(R^3)_z]_p\,H \qquad (I),$$

wobei
x jeden beliebigen Zahlenwert von 0,04 bis 0,4,
y jeden beliebigen Zahlenwert von 0,04 bis 0,92
und
z jeden beliebigen Zahlenwert von 0,04 bis 0,92

annehmen kann, mit der Bedingung, daß $(x+y+z) \leqq 2$ ist,

p für eine ganze Zahl von 100 bis 100.000 steht,

$R^1$ den Rest

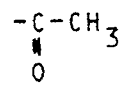

bedeutet,

$R^2$ den Rest

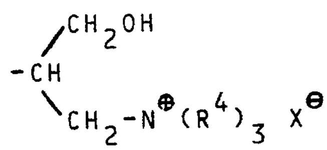

oder

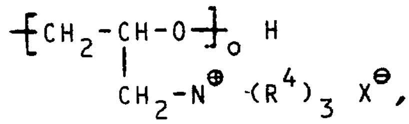

mit $R^4$ gleich $C_1$ bis $C_4$-Alkyl, X gleich Cl, Br, J, $CH_3SO_4$ und o gleich einer ganzen Zahl von 1 bis 5, darstellt und

$R^3$ den Rest

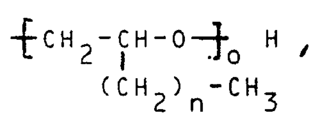

mit n gleich einer ganzen Zahl von 5 bis 20 und o gleich einer ganzen Zahl von 1 bis 5,

bedeutet.

## Claims

1. Cosmetic composition for treating hair and skin, characterised in that it is an aqueous or aqueous-alcoholic preparation containing from 0.05 to 10 percent by weight of a macromolecular, surface-active, quaternary N-substituted compound derived from Chitosan of the general formula (I)

   $HO\ [C_6H_{11-x-y-z}NO_4\ (R^1)_x\ (R^2)_y\ (R^3)_z]_p\ H$ (I),

   wherein x can take any numerical value from 0.04 to 0.4,
   y can take any numerical value from 0.04 to 0.92 and z can take any numerical value from 0.04 to 0.92, provided that $(x+y+z) \leqq 2$,
   p stands for a whole number from 100 to 100.000,
   $R^1$ is the residue

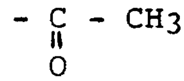

$R^2$ is the residue

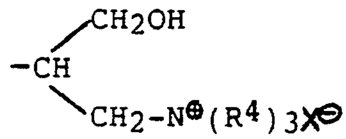

or

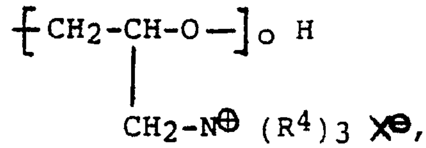

with $R^4$ equal to $C_1$ to $C_4$-alkyl, X equal to Cl, Br, I, $CH_3SO_4$ and o equal to a whole number from 1 to 5 and $R^3$ is the residue

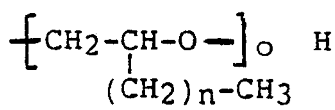

with n equal to a whole number from 5 to 20 and o equal to a whole number from 1 to 5.

2. Composition according to claim 1, characterised in that it is a solution, cream, gel, dispersion or emulsion.

3. Composition according to claim 1 or 2, characterised in that the pH-value is from 2 to 11.

4. Composition according to any one of claims 1 to 3, characterised in that it additionally contains a known filmforming, synthetic or natural cosmetic polymer.

5. Composition according to any one of claims 1 to 4, characterised in that it additionally contains at least one cosmetic dye in a concentration of 0.01 to 2.0 weight % and is in the form of a colour strengthener or tone strengthener.

6. Composition according to any one of claims 1 to 5, characterised in that it contains an aqueous or aqueous-alcoholic preparation as a cosmetic base, which is mixed with a propellant liquefied under pressure, charged into a pressure container and is in the form of aerosol foam.

7. Composition according to any one of the claims 1 to 6, characterised in that it is in the form of a shampoo and also contains at least one cationic, nonionic, amphoteric or anionic surfactant.

8. Medium according to claim 7, characterised in that the surfactant has a concentration of between 3 and 50 weight % and a pH-value of between 3 and 9.

9. Macromolecular, surface active, quaternary N-substituted compound derived from Chitosan of the

general formula (I)

HO $[C_6H_{11-x-y-z}NO_4(R^1)_x(R^2)_y(R^3)_z]_p$ H (I),

wherein x can take any numerical value from 0.04 to 0.4,
y can take any numerical value from 0.04 to 0.92 and z can take any numerical value from 0.04 to 0.92, provided that $(x+y+z)$ is $\leqq 2$,
p stands for a whole number from 100 to 100.000,
$R^1$ means the residue

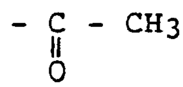

$R^2$ is the residue

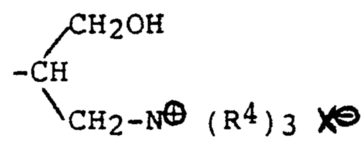

or

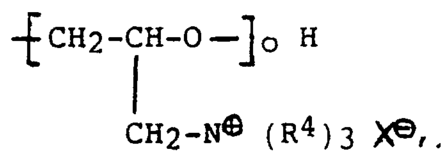

with $R^4$ equal to $C_1$ to $C_4$ - alkyl, X is equal to Cl, Br, I, $CH_3SO_4$ and o is equal to a whole number from 1 to 5, and $R^3$ is the residue

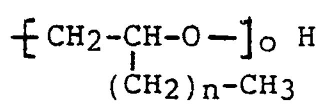

with n equal to a whole number from 5 to 20 and o equal to a whole number from 1 to 5.

**Revendications**

**1.** Produit cosmétique pour traiter les cheveux et la peau, caractérisé en ce qu'il contient, dans une préparation aqueuse ou aqueuse-alcoolique un composé macromoléculaire, tensio-actif, quaternaire, dérivé de la chitosane, substitué en N, de la formule générale (I)

HO $[C_6H_{11-x-y-z}NO_4(R^1)_x(R^2)_y(R^3)_z]_p$ H (I),

dans laquelle x peut prendre n'importe quelle valeur numérique comprise entre 0,04 et 0,4, y n'importe quelle valeur numérique comprise entre 0,04 et 0,92, z n'importe quelle valeur numérique entre 0,04 et 0,92, à condition que $(x+y+z) \leqq 2$, p est un nombre entier compris entre 100 et 100 000,

$R^1$ représente le radical

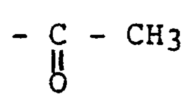

$R^2$ représente le radical

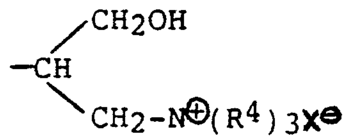

ou

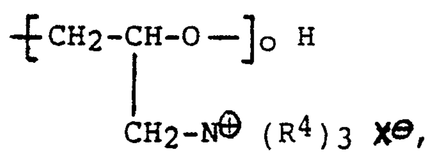

avec $R^4$ étant un alcoyle à 1 à 4 atomes de C, X étant Cl, Br, I, $CH_3SO_4$ et o étant égal à un nombre entier compris entre 1 et 5 et

$R^3$ représente le radical

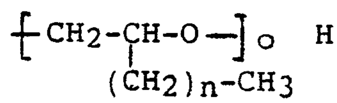

avec n égal à un nombre entier compris entre 5 et 20 et o égal à un nombre entier compris entre 1 et 5.

**2.** Produit selon la revendication 1, caractérisé en ce qu'il se présente sous forme de solution, crème, gel, dispersion ou émulsion.

**3.** Produit selon la revendication 1 ou 2, caractérisé en ce que le pH est compris entre 2 et 11.

**4.** Produit selon l'une des revendications 1 à 3, caractérisé en ce qu'il contient en plus un polymère cosmétique connu, synthétique ou naturel, filmogène.

**5.** Produit selon l'une des revendications 1 à 4, caractérisé en ce qu'il contient en plus au moins un colorant cosmétique en une concentration comprise entre 0,01 et 2,0 % en poids et qu'il se présente sous la forme d'un renforçateur de couleur ou d'un renforçateur de nuance.

**6.** Produit selon l'une des revendications 1 à 5, caractérisé en ce qu'il contient en tant que base cosmétique une préparation aqueuse ou aqueusealcoolique, qui est mélangée à un produit moussant liquéfié sous pression, qui est transvasé dans un récipient sous pression et se présente sous la forme d'une mousse aérosol.

**7.** Produit selon l'une des revendications 1 à 6, caractérisé en ce qu'il se présente sous la forme d'un produit de lavage pour les cheveux et contient en plus au moins un agent tensio-actif cationique, non-ionique, amphotère ou anionique.

**8.** Produit selon la revendication 7, caractérisé en ce qu'il contient l'agent tensio-actif en une concentration comprise entre 3 et 50 % en poids et qu'il a un pH compris entre 3 et 9.

**9.** Composé macromoléculaire, tensio-actif, quaternaire, dérivé du chitosane, substitué en N, de la formule générale (I)

$HO\ [C_6H_{11-x-y-z}\ NO_4\ (R^1)_x\ (R^2)_y\ (R^3)_z]_p\ H$ (I),

dans laquelle x peut prendre n'importe quelle valeur numérique comprise entre 0,04 et 0,4, y n'importe quelle valeur numérique comprise entre 0,04 et 0,92 et z n'importe quelle valeur numérique entre 0,04 et 0,92, à la condition que (x + y + z) soit < ou égal à 2, p est un nombre entier entre 100 et 100 000, $R^1$ représente le radical

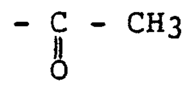

$R^2$ représente le radical

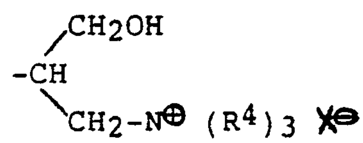

ou

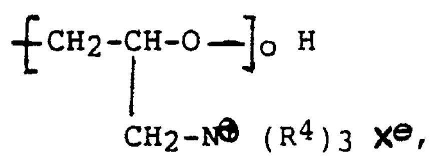

avec $R^4$ étant un alcoyle à 1 à 4 atomes de C, X étant Cl, Br, I, $CH_3SO_4$ et o étant égal à un nombre entier compris entre 1 et 5 et

$R^3$ représentant le radical

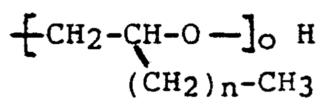

avec n égal à un nombre entier compris entre 5 et 20 et o égal à un nombre entier compris entre 1 et 5.